Europäisches Patentamt

European Patent Office

Office européen des brevets

(19)

(11) **EP 0 813 857 B1**

(12) **FASCICULE DE BREVET EUROPEEN**

(45) Date de publication et mention
de la délivrance du brevet:
**02.12.1998 Bulletin 1998/49**

(51) Int. Cl.⁶: **A61K 7/00**, A61K 7/42

(21) Numéro de dépôt: **97401206.4**

(22) Date de dépôt: **30.05.1997**

(54) **Nouveau procédé de préparation de compositions uv-filtrantes, compositions susceptibles d'être obtenues par ce procédé et applications**

Verfahren zur Herstellung von UV-Filtern enthaltende Zusammensetzungen, Zusammensetzung und Verwendung

Method of preparation of UV filter containing compositions, composition and uses

(84) Etats contractants désignés:
**DE ES FR GB IT**

(30) Priorité: **21.06.1996 FR 9607775**

(43) Date de publication de la demande:
**29.12.1997 Bulletin 1997/52**

(73) Titulaire: **L'OREAL**
**75008 Paris (FR)**

(72) Inventeurs:
• **Hansenne, Isabelle**
**75017 Paris (FR)**

• **de Chabannes, Karine**
**45000 Orleans (FR)**
• **Vernaire, Sandrine**
**75015 Paris (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**EP-A- 0 552 024          EP-A- 0 685 228**
**WO-A-96/14076**

EP 0 813 857 B1

**Description**

La présente invention se rapporte à un procédé de préparation de compositions destinées à la photoprotection de la peau et/ou des cheveux contre le rayonnement ultraviolet, en particulier le rayonnement solaire, présentant un SPF et une rémanence à l'eau améliorés. L'invention se rapporte également aux compositions susceptibles d'être obtenues par ce procédé (compositions ci-après dénommées plus simplement compositions antisolaires) et à leurs applications dans le domaine cosmétique et/ou dermatologique.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques et/ou dermatologiques destinées à la photoprotection (UV-A et/ou UV-B) de la peau ont été proposées à ce jour.

Ces compositions antisolaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée huileuse) ou eau-dans-huile (phase aqueuse dispersée dans une phase huileuse continue), qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire (SPF) s'exprimant mathématiquement par le rapport du temps d'irradiation nécessaire pour atteindre le seuil érythématogène avec le filtre UV au temps nécessaire pour atteindre le seuil érythématogène sans filtre UV). Dans de telles émulsions, les filtres hydrophiles sont présents dans la phase aqueuse et les filtres lipophiles sont présents dans la phase grasse.

Les émulsions huile-dans-eau sont, d'une manière générale, plus appréciées par le consommateur que les émulsions eau-dans-huile, en raison notamment de leur toucher agréable (voisin de l'eau) et de leur présentation sous forme de lait ou de crème non gras ; cependant, elles perdent également plus facilement leur efficacité en protection UV dès lors qu'elles viennent en contact avec l'eau ; en effet, les filtres hydrophiles, acides notamment, disparaissent à l'eau, par baignade en mer ou en piscine, sous la douche ou lors de la pratique de sports nautiques ; ainsi, les compositions solaires qui les contiennent, seuls ou associés aux filtres lipophiles, n'apportent plus la protection initiale recherchée dès lors que le substrat (peau ou cheveu) sur lequel elles ont été appliquées vient en contact avec l'eau, cette perte en indice de protection par élimination à l'eau du filtre hydrophile étant d'autant plus marquée que l'association filtrante lipophile-hydrophile, présente dans la composition, est synergique au niveau du facteur de protection solaire.

On peut disposer de compositions antisolaires présentant des SPF et une résistance à l'eau améliorés en mettant en oeuvre des émulsions eau-dans-huile. En effet, un filtre hydrophile est plus rémanent à l'eau au sein d'une émulsion eau-dans-huile qu'au sein d'une émulsion huile-dans-eau. Cependant, comme il a été indiqué plus haut, de telles compositions ne donnent pas encore entièrement satisfaction dans la mesure où elles laissent après application une impression de gras particulièrement désagréable pour l'utilisateur.

On a également proposé d'améliorer le SPF des émulsions, en particulier huile-dans-eau, antisolaires contenant un ou plusieurs filtres hydrophiles en associant ces derniers à certains filtres lipophiles bien particuliers, comme par exemple décrit dans la demande EP-A-0 685 228 au nom de la Demanderesse. Cependant, selon cette technique, les améliorations du SPF obtenues reposent sur l'association synergique de filtres hydrophiles et lipophiles spécifiques (comme l'acide benzène 1,4-di(3-méthylidène-10-camphosulfonique) et l'$\alpha$-cyano-$\beta,\beta$-diphénylacrylate de 2-éthyl-héxyle dans le cas de EP-A-0 685 228) au sein d'émulsions huile-dans-eau classiques, de sorte que ces améliorations ne peuvent pas être obtenues de façon générale, c'est-à-dire quels que soient les filtres hydrophile(s) et lipophile(s) utilisés.

Ainsi, le besoin subsiste toujours quant à pouvoir disposer de compositions antisolaires comprenant à la fois au moins un filtre hydrophile et au moins un filtre lipophile, et dont le SPF serait élevé et stable dans le temps, quels que soient les filtres hydrophile(s) et/ou lipophile(s) utilisés.

La présente invention vise à la satisfaction d'un tel besoin, en proposant notamment un nouveau procédé de préparation de compositions antisolaires contenant au moins un filtre hydrophile et au moins un filtre lipophile, lesdites compositions, nouvelles en soi, présentant, à système filtrant semblable [filtre(s) hydrophile(s) + filtre(s) lipophile(s)], des propriétés améliorées par rapport aux compositions de l'art antérieur à supports huile-dans-eau ou eau-dans-huile classiques, notamment au niveau des SPF obtenus et de leur stabilité, en particulier la rémanence à l'eau.

Le procédé classique de préparation des émulsions en général, et des émulsions antisolaires comprenant des filtres lipophiles et hydrophiles en particulier, est bien connu.

Un tel procédé consiste à préparer, à chaud (généralement aux alentours de 80°C) et sous agitation, une phase grasse A contenant au moins un émulsionnant, éventuellement un coémulsionnant, au moins une huile, le ou les filtres lipophiles et éventuellement un ou plusieurs autres corps gras. On prépare séparément une phase aqueuse B comprenant le ou les filtres hydrophiles et de l'eau, éventuellement un ou plusieurs hydratants, cette phase étant portée à la même température que la phase grasse, et homogénéisée par simple agitation. On mélange ensuite ces deux phases A et B sous agitation afin de réaliser une émulsion qu'on laisse refroidir également sous agitation. En dernier lieu, on peut éventuellement rajouter une troisième phase C, qui comprend un épaississant.

On réalise ainsi des émulsions antisolaires classiques, soit de type huile-dans-eau soit de type eau-dans-huile selon la nature du HLB (balance lipophile-hydrophile) du système émulsionnant choisi.

Un tel procédé classique ne permet pas d'aboutir à des compositions antisolaires présentant des performances comparables à celles proposées selon la présente invention.

La Demanderesse vient de trouver, après de longues recherches sur la question, un nouveau procédé de préparation d'émulsions antisolaires permettant d'aboutir à des compositions nouvelles à propriétés améliorées, notamment au niveau des SPF qui leur sont attachés. Plus précisément encore, on a trouvé que la préparation préalable d'une phase comprenant l'ensemble des filtres hydrophiles et lipophiles, puis l'ajout de cette phase dans une émulsion déjà réalisée, conduisait à des compositions particulièrement performantes tant au niveau de la valeur-même du SPF qu'au niveau de la stabilité dans le temps de ce dernier, et en particulier de sa rémanence à l'eau.

Cette découverte est à l'origine de la présente invention.

Ainsi, selon un premier objet de la présente invention, il est maintenant proposé un nouveau procédé de préparation d'une composition comprenant, dans un support cosmétiquement et/ou dermatologiquement acceptable de type mixte huile-eau, au moins un filtre lipophile et au moins un filtre hydrophile, et qui est caractérisé par le fait qu'il consiste essentiellement à introduire, dans une émulsion huile-dans-eau obtenue par mélange entre i) une phase aqueuse A et ii) une phase huileuse $H_1$ comprenant un émulsionnant H/E, une phase huileuse $H_2$ comprenant un émulsionnant E/H, ledit filtre lipophile et ledit filtre hydrophile, lesdites phases huileuse $H_1$ et $H_2$ étant incompatibles entre elles.

Un tel procédé permet d'obtenir des compositions comprenant simultanément un filtre hydrophile et un filtre lipophile et qui présentent un SPF particulièrement élevé, et en tout cas supérieur à celui des compositions contenant dans un support différent le même système filtrant.

Un autre objet de l'invention concerne de nouvelles compositions cosmétiques et/ou dermatologiques, susceptibles d'être obtenues par le procédé défini ci-dessus, et qui sont caractérisées par le fait qu'elles comprennent :

i) une phase aqueuse continue dispersante,

ii) une première phase huileuse $H_1$ dispersée discontinue,

iii) une deuxième phase huileuse $H_2$ dispersée discontinue,

iv) au moins un émulsionnant H/E,

v) au moins un émulsionnant E/H,

vi) au moins un filtre lipophile,

vii) au moins un filtre hydrophile,

lesdites première et deuxième phases huileuses $H_1$ et $H_2$ étant incompatibles entre elles, les globules constituant ladite phase huileuse $H_1$ ayant une taille moyenne différente des globules constituant ladite phase huileuse $H_2$.

Les compositions selon l'invention, outre leur remarquable efficacité en ce qui concerne la photoprotection de la peau et/ou des cheveux, présentent également une très bonne rémanence du SPF à l'eau. Autrement dit, les filtres hydrophiles qu'elles contiennent sont très peu entraînés par l'eau et les compositions restent efficaces même après une baignade en mer ou en piscine par exemple.

La présente invention a encore pour objet un procédé de traitement cosmétique pour la protection de la peau et/ou des cheveux contre le rayonnement ultraviolet, en particulier le rayonnement solaire, consistant à appliquer sur ces derniers une quantité efficace d'une composition telle que définie ci-dessus.

La présente invention a également pour objet l'utilisation de la composition telle que définie ci-dessus comme, ou pour la fabrication de, compositions cosmétiques pour la protection de la peau et/ou des cheveux contre le rayonnement ultraviolet, en particulier le rayonnement solaire.

EP 0 813 857 B1

D'autres caractéristiques, aspects et avantages de la présente invention apparaîtront à la lecture de la description détaillée qui va suivre.

La première étape du procédé selon l'invention consiste à préparer de façon classique une phase huileuse $H_1$. Cette dernière comprend au moins un émulsionnant H/E. Par émulsionnant H/E, on entend, au sens de la présente invention et dans le texte qui suit, tout composé ayant des propriétés émulsionnantes permettant de réaliser des émulsions huile-dans-eau. Comme émulsionnants H/E utilisables dans la présente invention, on peut citer les émulsionnants H/E habituellement utilisés dans la préparation des émulsions huile-dans-eau classiques tels que les esters d'acides gras et de polyéthylène glycol (PEG), les esters d'acides gras et de glycérol (stéarate de glycéryle) ou les esters d'acides gras et de sucre (stéarate de sorbitane), ainsi que leurs dérivés polyoxyéthylénés ou polyoxypropylénés, les éthers de sucre, les tensioactifs anioniques (alkylphosphate de K ou de Na), les alcools gras polyalcoxylés.

On peut également citer les polymères à chaîne grasse ayant des propriétés émulsionnantes et qui permettent de réaliser des émulsions huile-dans-eau tels que par exemple le copolymère acide acrylique / acrylate d'alkyles en C10-C30 vendu sous la dénomination commerciale 《 Pemulen TR-1 》 par la société Goodrich.

Un émulsionnant H/E particulièrement préféré selon la présente invention est le mélange de mono/distéarate de glycérol et de stéarate de polyéthylène glycol (100 OE) vendu sous la dénomination commerciale 《 Arlacel 165 》 par la société ICI.

L'émulsionnant H/E peut être présent dans la composition finale selon l'invention à une teneur pouvant aller de 0,1 % à 10 %, de préférence de 0,1 % à 5 % en poids, par rapport au poids total de la composition.

Cette première phase huileuse $H_1$ peut également comprendre un coémulsionnant destiné à donner de la consistance à l'émulsion. Les coémulsionnants peuvent être par exemple choisis parmi l'alcool stéarylique et l'acide stéarique.

La première phase huileuse comprend également un ou plusieurs corps gras, ces corps gras pouvant être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (comme le benzoate d'alcools en C12-C15 vendu sous la dénomination commerciale 《 Finsolv TN 》 par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Cette première phase huileuse $H_1$ est généralement préparée de façon classique en mélangeant les divers constituants sous simple agitation à chaud. Ce mélange peut se faire à une température de 80 °C environ par exemple.

De préférence, cette phase huileuse $H_1$ est exempte de tout filtre, en particulier de filtre liposoluble.

Une deuxième étape du procédé selon l'invention consiste à préparer de façon classique une phase aqueuse. Cette dernière comprend généralement un ou plusieurs hydratants et de l'eau. Elle peut également comprendre d'autres constituants hydrophiles habituellement utilisés dans les compositions cosmétiques et/ou dermatologiques.

Cette phase aqueuse est généralement préparée de façon classique en mélangeant les divers constituants sous simple agitation à chaud. Ce mélange peut se faire à une température de 80 °C environ par exemple.

De préférence, cette phase aqueuse est exempte de tout filtre, en particulier de filtre hydrosoluble.

Selon une troisième étape du procédé selon l'invention, essentielle, on réalise une deuxième phase huileuse $H_2$, séparément des phases aqueuse et huileuse $H_1$, cette phase $H_2$ comprenant au moins un émulsionnant E/H, au moins un filtre hydrophile et au moins un filtre lipophile.

Par émulsionnant E/H, on entend, au sens de la présente invention et dans le texte qui suit, tout composé ayant des propriétés émulsionnantes permettant de réaliser des émulsions eau-dans-huile.

Comme émulsionnants E/H utilisables dans la préparation de la phase huileuse $H_2$ du procédé selon la présente invention, on peut plus particulièrement citer les diméthicone copolyols et leurs esters tels que les diméthicone copolyols vendus sous les dénominations commerciales 《 Silicone $Q_2$ 5220 》, 《 Silicone DC 193 》 ou 《 Silicone $Q_2$ 3225C 》 par Dow Corning. Une diméthicone particulièrement préférée pour la mise en oeuvre de la présente invention est celle vendue sous la dénomination commerciale 《 Silicone $Q_2$ 3225C 》 par Dow Corning.

L'émulsionnant E/H est généralement présent dans la composition finale selon l'invention à une teneur pouvant aller de 0,1 à 10 %, de préférence de 0,1 à 5 %, en poids, par rapport au poids total de la composition.

Une caractéristique essentielle de la présente invention est la présence dans cette phase $H_2$ d'un ou de plusieurs filtres solaires actifs dans l'UVA et/ou l'UVB (absorbeurs), hydrophiles. Ce ou ces filtres hydrophiles peuvent être notamment choisis parmi les dérivés de la benzophénone, les dérivés de l'acide p-aminobenzoïque, les dérivés du camphre ou encore parmi les dérivés de benzimidazole.

4

Comme filtres hydrophiles particulièrement utilisables dans la présente invention, on peut citer l'acide benzène 1,4-di(3-méthylidéne-10-camphosulfonique) et l'acide 2-phénylbenzimidazole-5-sulfonique vendu sous la dénomination commerciale 《Eusolex 232 》par la société Merck.

Le ou les filtres hydrophiles peuvent être présents dans la composition finale selon l'invention à une teneur pouvant varier de 0,1 à 20 %, de préférence de 0,2 à 10 %, en poids, par rapport au poids total de la composition.

Selon une autre caractéristique essentielle de la présente invention, la phase huileuse $H_2$ comprend également au moins un filtre solaire actif dans l'UVA et/ou l'UVB lipophile. Les filtres lipophiles convenant particulièrement bien à la présente invention peuvent être choisis parmi les dérivés du dibenzoyméthane, les dérivés de benzimidazole, les dérivés cinnamiques, les dérivés salicyliques, les dérivés du camphre, les dérivés de triazine, les dérivés de la benzophénone, les dérivés de $\beta,\beta$-diphénylacrylate, les dérivés de l'acide p-aminobenzoïque, les polymères filtres et les silicones filtres décrits dans les demandes WO-93/04665 et WO-94/06404. D'autres exemples de filtres organiques sont donnés dans la demande de brevet EP-A 0 487 404.

Comme filtres lipophiles particulièrement utilisables dans la présente invention, on peut citer le 4-tert-butyl-4'-méthoxydibenzoylméthane vendu sous la dénomination commerciale 《Parsol 1789 》par la société Givaudan et l'$\alpha$-cyano-$\beta,\beta$-diphénylacrylate de 2-éthylhexyle vendu sous la dénomination commerciale 《Uvinul N 539 》par la société BASF.

Le ou les filtres lipophiles peuvent être présents dans la composition finale selon l'invention à une teneur pouvant varier de 0,5 à 30 %, de préférence de 0,5 à 20 %, en poids, par rapport au poids total de la composition.

De préférence, la phase huileuse $H_2$ comprend tous les filtres UVA et/ou UVB, hydrophile(s) et lipophile(s) que l'on souhaite incorporer dans la composition finale selon l'invention.

Cette phase huileuse $H_2$ comprend également un ou plusieurs corps gras choisi parmi ceux cités ci-dessus pour la préparation de la phase huileuse $H_1$, de telle sorte que les phases huileuses $H_1$ et $H_2$ soient incompatibles dans la composition. De préférence, ce corps gras est une huile de silicone, volatile telle que les cyclométhicones ou non volatile telle que les diméthicones. De préférence encore, on utilise dans la présente invention une huile de silicone volatile comme par exemple les cyclométhicones vendues sous les dénominations commerciales 《 DC 245 Fluid 》ou 《DC 246 Fluid 》par Dow Corning.

Cette huile de silicone peut être présente dans la composition finale selon l'invention à une teneur pouvant aller de 1 à 20 %, de préférence de 2 à 10 %, en poids, par rapport au poids total de la composition.

La quatrième étape du procédé selon l'invention consiste à réaliser une émulsion huile-dans-eau en introduisant sous agitation classique la première phase huileuse $H_1$ dans la phase aqueuse préparée à la deuxième étape.

La cinquième étape du procédé selon l'invention consiste à incorporer dans l'émulsion ainsi réalisée la phase huileuse $H_2$. Cette étape peut se faire sous agitation simple sans appareillage particulier. Après mélange, on laisse refroidir la composition à température ambiante.

Les compositions finales obtenues selon le procédé conforme à l'invention comprennent alors une phase aqueuse continue dispersante et deux phases huileuses $H_1$ et $H_2$ discontinues dispersées, ces deux phases huileuses étant incompatibles entre elles et donc séparées, les globules constituant la phase huileuse $H_1$ ayant une taille moyenne différente de ceux constituant la phase huileuse $H_2$, ainsi que les filtres lipophile(s) et hydrophile(s).

On distingue ainsi dans les compositions conformes à l'invention la coexistence de deux populations de globules d'huile de tailles différentes, à savoir une première population constituée de globules d'huile dont la taille varie généralement de 1 à 10 µm, et une deuxième population formée de globules d'huile beaucoup plus fins, de taille généralement inférieure à 1 µm.

Selon toutes vraissemblances, les gros globules correspondent à la phase huileuse $H_2$, et les petits globules à la phase huileuse $H_1$.

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium, les silicones. De tels nanopigments d'oxydes métalliques, enrobés ou non enrobés, sont en particulier décrits dans les demande de brevets EP-A- 0 518 772 et EP-A- 0 518 773.

Les nanopigments peuvent être présents dans la composition finale selon l'invention à une teneur pouvant varier de 0,1 à 20 %, de préférence de 0,2 à 10 %, en poids, par rapport au poids total de la composition.

Les compositions selon l'invention peuvent également contenir des épaississants.

Les épaississants peuvent être choisis notamment parmi les acides polyacryliques réticulés, les acides polyacryliques à chaîne grasse, les gommes de guar et celluloses modifiées ou non telles que la gomme de guar hydroxypropylée, la méthylhydroxyéthylcellulose et l'hydroxypropylméthyl cellulose.

Les ou les épaississants peuvent être présents dans la composition finale selon l'invention à une teneur pouvant aller de 0,1 à 10 %, de préférence de 0,1 à 5 %, en poids, par rapport au poids total de la composition.

Ce ou ces épaississants peuvent être introduits dans la composition selon l'invention soit avant, soit après l'étape 5°) d'incorporation de la phase huileuse H$_2$.

Les compositions selon l'invention peuvent également contenir des agents de bronzage et/ou de brunissage artificiels de la peau (agents autobronzants), tels que par exemple de la dihydroxyacétone (DHA).

Les compositions conformes à la présente invention peuvent comprendre en outre des adjuvants cosmétiques et/ou dermatologiques classiques notamment choisis parmi les solvants organiques, les adoucissants, les antioxydants, les agents anti radicaux libres, les opacifiants, les stabilisants, les émollients, les $\alpha$-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

La composition cosmétique et/ou dermatologique de i'invention peut être utilisée comme composition protectrice de l'épiderme humain ou des cheveux contre les rayons ultraviolets, comme composition antisolaire ou comme produit de maquillage.

Des exemples concrets, mais nullement limitatifs, illustrant l'invention, vont maintenant être donnés.

### EXEMPLE 1:

On a réalisé les trois émulsions suivantes : une émulsion (I), conforme à l'invention et préparée selon le procédé de l'invention; une émulsion (II), comprenant le même système filtrant que l'émulsion (I) mais préparée selon un procédé classique de préparation d'émulsion huile-dans-eau; et l'émulsion (III), comprenant le même système filtrant que les émulsions (I) et (II), mais qui est une émulsion eau-dans-huile.

Les compositions des formules sont données ci-après (les quantités sont exprimées en pourcentage de poids par rapport au poids total de la composition) :

### Emulsion (I) : (invention)

| $A_1$ | |
|---|---|
| - acide stéarique | 1,5 % |
| - mélange mono/distéarate de glycérol/stéarate de polyéthylène glycol (100 0E) vendu sous la dénomination commerciale 〈〈 Arlacel 165 〉〉 par ICI (émulsionnant H/E) | 1,5 % |
| - alcool stéarylique | 0,5 % |
| - conservateur | qs |
| - copolymère vinylpyrrolidone/eicosine vendu sous la dénomination commerciale 〈〈 Antaron V220 〉〉par ISP | 1 % |

| $A_2$ : | |
|---|---|
| - triéthanolamine | 0,45 % |

| $B_1$ : | |
|---|---|
| - 4-tert-butyl-4'-méthoxydibenzoylméthane vendu sous la dénomination commerciale 〈〈 Parsol 1789 〉〉 par Givaudan (filtre lipophile) | 2 % |

(suite)

| $B_1$ : | |
|---|---|
| - $\alpha$-cyano-$\beta,\beta$-diphénylacrylate de 2-éthylhexyle vendu sous la dénomination commerciale ⟨⟨ Uvinul N 539 ⟩⟩ par BASF (filtre lipophile) | 10 % |

| $B_2$ : | |
|---|---|
| - polydiméthylsiloxane vendu sous la dénomination commerciale ⟨⟨ Silicone $Q_2$3225C ⟩⟩ par Dow Corning (émulsionnant E/H) | 1 % |
| - cyclohexa diméthylsiloxane vendu sous la dénomination commerciale ⟨⟨ DC 246 Fluid ⟩⟩ par Dow corning | 5 % |

| $B_3$ : | |
|---|---|
| - acide benzène 1,4-di(3-méthylidène-10-camphosulfonique) (filtre hydrophile) | 1,5 % (0,5 % MA) |
| - triéthanolamine | 0,26 % |

| $C$ : | |
|---|---|
| - hexa decyl phosphate de K vendu sous la dénomination commerciale ⟨⟨ Amphisol K ⟩⟩ par Roche | 1 % |

| $D$ : | |
|---|---|
| - nanopigment d'oxyde de titane vendu sous la dénomination commerciale ⟨⟨ MT100T ⟩⟩ par Tayca | 5 % |

| $E$ : | |
|---|---|
| - hydratants | 15 % |
| - conservateur | qs |
| - séquestrant | 0,1 % |

| F : | |
|---|---|
| - acide polyacrylique vendu sous la dénomination commerciale 〈〈 Carbopol 980 〉〉 par Goodrich | 0,3 % |
| - benzoate d'alcools en $C_{12}/C_{15}$ vendu sous la dénomination commerciale 〈〈 Finsolv TN 〉〉 par Stéarineries Dubois | 2 % |

| G : | |
|---|---|
| - triethanolamine | 0,3 % |

| J : | |
|---|---|
| - eau purifiée | qsp 100 % |

**Mode opératoire :**

a) <u>Préparation des phases huileuses $H_1$ et $H_2$ et de la phase aqueuse selon l'invention :</u>

    1°) Préparation de la phase $H_1$ selon l'invention (phase huileuse contenant l'émulsionnant H/E) :
        On fait fondre, à 80 °C et sous agitation, $A_1$. Puis on ajoute $A_2$.

    2°) Préparation de la phase aqueuse :
        On dissout E dans J à 80 °C. Puis on ajoute C.

    3°) Préparation de la phase $H_2$ selon l'invention (phase huileuse comprenant les filtres et l'émulsionnant E/H) :
        On porte $B_1$ à 70 °C. Puis, on introduit lentement $B_1$ dans $B_2$ sous agitation turbine (obtention d'un mélange blanc crémeux). On refroidit jusqu'à 40 °C sous agitation. Enfin, on incorpore très lentement $B_3$ dans le mélange obtenu.

b) <u>Fabrication de la composition finale :</u>

    Sous agitation, on introduit à 75 °C la phase huileuse $H_1$ dans la phase aqueuse. Puis, vers 70 °C, on introduit D. Vers 60 °C, on introduit F (préalablement préparée par dispersion à froid du Carbopol 980 dans le Finsolv TN sous agitation). Puis, on neutralise par addition de G. On refroidit à 40 °C. Enfin, on introduit sous agitation dans l'émulsion $H_1$/E ainsi obtenue la phase huileuse $H_2$. On refroidit à 20 °C.

**Emulsion (II) : (comparative : émulsion classique huile-dans-eau)**

    La composition de l'émulsion (II) est la même que celle de l'émulsion (I) mais ne comprend pas la phase $B_2$.

**Mode opératoire :**

    La phase huileuse ($A_1 + A_2$) est préparée comme dans l'exemple 1 ; puis on y introduit ensuite $B_1$.
    Séparément, on dissout E dans J à 80 °C dans une cuve ; puis on ajoute C puis $B_3$. A 75 °C, sous agitation, on ajoute dans cette phase aqueuse la phase huileuse ($A_1 + A_2 + B_1$), préparée ci-avant, pour obtenir une émulsion H/E.
    Vers 70°C, on introduit D. Vers 60 °C, on introduit F (préalablement préparée par dispersion à froid du Carbopol 980 dans le Finsolv TN sous agitation). Puis on neutralise par addition de G. Enfin, on refroidit à 20°C.

**Emulsion (III) : (comparative : émulsion classique eau-dans-huile)**

| $A_1$ : | |
|---|---|
| - polyméthyllauryl/methylsiloxane OE et OP vendu sous la dénomination commerciale 《DC $Q_2$ 5200 》par Dow corning | 2,5 % |
| - cyclohexa diméthylsiloxane vendu sous la dénomination commerciale 《DC 246 Fluid 》par Dow corning | 8 % |
| - palmitate d'isopropyle | 7 % |
| - poly dimethylsiloxane à groupements behenate vendu sous la dénomination commerciale 《Mirasil wax - B 》par Rhône-Poulenc | 1 % |

| $A_2$ : | |
|---|---|
| - 4-tert-butyl-4'-méthoxydibenzoylméthane vendu sous la dénomination commerciale 《Parsol 1789 》par Givaudan | 2 % |
| - $\alpha$-cyano-$\beta,\beta$-diphénylacrylate de 2-éthylhexyle vendu sous la dénomination commerciale 《Uvinul N 539 》par BASF | 10 % |

| $B$ : | |
|---|---|
| - NaCl | 2 % |
| - hydratants | 8 % |

| $C$ : | |
|---|---|
| - acide benzène 1,4-di(3-méthylidène-10-camphosulfonique) | 1,5 % (0,5 % MA) |
| - triethanolamine | 0,26 % |

| $D$ : | |
|---|---|
| - nanopigment d'oxyde de titane vendu sous la dénomination commerciale 《MT100T 》par Tayca | 5 % |

| E : | |
|---|---|
| - eau purifiée | qsp 100 % |

**Mode opératoire :**

On porte $A_1$ sans la silicone 《 DC 246 Fluid 》 à 60 °C. On refroidit à 40 °C, puis on ajoute la silicone 《 DC 246 Fluid 》. On porte ensuite $A_2$ à 60 °C. On refroidit à 40 °C. On mélange $A_1$ et $A_2$. Séparément, on chauffe le mélange (B + E) à 60 °C. On le refroidit à 40 °C, puis, sous agitation, on l'introduit dans la phase $(A_1 + A_2)$ pour réaliser l'émulsion. On introduit ensuite D sous agitation dans cette émulsion. Puis, on introduit C sous agitation. Enfin, on laisse refroidir à 25 °C.

**Evaluation du SPF des compositions obtenues :**

Pour chacune des formulations (I), (II) et (III) ainsi préparées, on a ensuite déterminé le facteur de protection solaire (SPF) qui lui est attaché. La mesure du facteur de protection solaire a été effectuée selon la méthode suivante *(in vivo)*: on a appliqué ces formulations, à raison de 2 mg de produit / $cm^2$ de peau, sur le dos de 5 modèles humains, puis on a soumis simultanément les zones protégées et les zones non protégées de peau à l'action d'un simulateur solaire commercialisé sous le nom de "Xénon Multiport WG 320-UG 11" ; le facteur de protection solaire (SPF) a été alors calculé mathématiquement par le rapport du temps d'irradiation qui a été nécessaire pour atteindre le seuil érythématogène avec le filtre UV (zone protégée) au temps qui a été nécessaire pour atteindre le seuil érythématogène sans filtre UV (zone non protégée).

On a ensuite fait prendre deux bains d'eau aux 5 modèles, chaque bain durant 20 minutes, les deux bains étant espacés d'une durée de 20 minutes.

Puis le SPF a de nouveau été déterminé selon le même protocole que ci-dessus.

Les résultats en SPF et avant et après les deux bains sont donnés dans le tableau (I) ci-dessous :

Tableau (I)

| | SPF avant bain | SPF après bain |
|---|---|---|
| Emulsion (I) (invention) | 59,4 | 38,9 |
| Emulsion (II) (comparative) | 34,0 | 10,5 |
| Emulsion (III) (comparative) | 31,9 | 17,4 |

Ces résultats montrent clairement que, à système filtrant identique, l'émulsion (I), conforme à la présente invention, présente un SPF qui est d'une part particulièrement élevé et d'autre part qui résiste remarquablement à l'eau. En particulier, même après deux bains, le SPF de l'émulsion selon l'invention est beaucoup plus élevé que celui observé pour une émulsion eau-dans-huile présentant un système filtrant identique (émulsion (III)).

**EXEMPLE 2 :**

Cet exemple a pour but de comparer deux émulsions rigoureusement identiques en composition, mais préparées selon deux procédés différents.

Dans cet exemple, on a ainsi repris l'émulsion (I) de l'exemple 1 et l'émulsion (II) de l'exemple 1, l'émulsion (II) comprenant cette fois la phase $B_2$ : cette émulsion est appelée ci-après émulsion (II'). Les émulsions (I) et (II') ont donc exactement la même composition. On a fabriqué l'émulsion (I) selon le procédé conforme à l'invention décrit dans l'exemple 1. L'émulsion (II') a été fabriquée selon le procédé classique de préparation de l'émulsion (II) de l'exemple 1 (émulsion H/E), en incorporant la phase $B_2$ dans la phase huileuse au moment de la préparation de cette dernière.

On a ensuite évalué le SPF in vitro pour chacune de ces deux émulsions (I) et (II'). Celui-ci a été déterminé en utilisant la méthode *in vitro* décrite par B.L. DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les résultats (valeur moyenne correspondant à cinq essais) sont regroupés dans le tableau (II) ci-dessous :

Tableau (II)

|  | Emulsion (I) (invention) | Emulsion (II') (comparative) |
|---|---|---|
| SPF | 70,4 | 56,2 |

Ces résultats montrent clairement, qu'à composition rigoureusement identique, l'émulsion fabriquée selon le procédé conforme à l'invention (émulsion (I)) présente un SPF beaucoup plus élevé que celle fabriquée par un procédé classique de préparation d'émulsion H/E (émulsion (II')).

**EXEMPLE 3 :**

On donne ci-après un autre exemple concret d'une composition antisolaire sous la forme d'un gel-crème.

| $A_1$ | |
|---|---|
| - copolymère acide acrylique / acrylate d'alkyles en C10-C30 vendu sous la dénomination commerciale 〈〈 Pemulen TR-1 〉〉 par la société Goodrich | 0,4 % |

| $A_2$ : | |
|---|---|
| - triéthanolamine | 0,4 % |

| $B_1$ : | |
|---|---|
| - 4-tert-butyl-4'-méthoxydibenzoylméthane vendu sous la dénomination commerciale 〈〈 Parsol 1789 〉〉 par Givaudan | 2 % |
| - $\alpha$-cyano-$\beta,\beta$-diphénylacrylate de 2-éthylhexyle vendu sous la dénomination commerciale 〈〈 Uvinul M 539 〉〉 par BASF | 10 % |

| $B_2$ : | |
|---|---|
| - polydiméthylsiloxane vendu sous la dénomination commerciale 〈〈 Silicone $Q_2$3225C 〉〉 par Dow Corning | 1 % |
| - cyclohexa diméthylsiloxane vendu sous la dénomination commerciale 〈〈 DC 246 Fluid 〉〉 par Dow corning | 5 % |

| $B_3$ : | |
|---|---|
| - acide benzène 1,4-di(3-méthylidène-10-camphosulfonique) | 1,5 % (0,5 % MA) |
| - triethanolamine | 0,26 % |

| $C$ : | |
|---|---|
| - nanopigment d'oxyde de titane vendu sous la dénomination commerciale ⟨⟨ MT100T ⟩⟩ par Tayca | 5 % |

| $D$ : | |
|---|---|
| - hydratants | 8 % |
| - conservateur | qs |
| - séquestrant | 0,1 % |

| $E$ : | |
|---|---|
| - benzoate d'alcools en $C_{12}/C_{15}$ vendu sous la dénomination commerciale ⟨⟨ Finsolv TN ⟩⟩ par Stéarineries Dubois | 4 % |

| $F$ : | |
|---|---|
| - eau purifiée | qsp 100 % |

Ce gel-crème a été préparé de la façon suivante : on a d'abord dispersé le ⟨⟨ Pemulen TR1 ⟩⟩ neutralisé par la triéthanolamine dans le ⟨⟨ Finsolv TN ⟩⟩ (préparation de la phase $H_1$ selon l'invention). Puis on a préparé la phase aqueuse en dissolvant D dans F. On a ensuite préparé la phase $H_2$ selon l'invention de la même manière que dans l'exemple 1 ($B_1 + B_2 + B_3$). La phase $H_1$ a été introduite dans la phase aqueuse sous agitation. Vers 70 °C, on a ajouté C. On a refroidi à 40 °C et on a introduit la phase $H_2$ telle que préparée ci-dessus. Pour finir, on a refroidi à 20 °C.

Ce gel-crème présente un SPF particulièrement élevé et très résistant à l'eau.

**Revendications**

1. Procédé de préparation d'une composition comprenant, dans un support cosmétiquement et/ou dermatologiquement acceptable de type mixte huile-eau, au moins un filtre lipophile et au moins un filtre hydrophile, caractérisé par le fait qu'il consiste essentiellement à introduire, dans une émulsion huile-dans-eau obtenue par mélange entre i) une phase aqueuse A et ii) une phase huileuse $H_1$ comprenant un émulsionnant H/E, une phase huileuse $H_2$

comprenant un émulsionnant E/H, ledit filtre lipophile et ledit filtre hydrophile, lesdites phases huileuse $H_1$ et $H_2$ étant incompatibles entre elles.

2. Procédé selon la revendication 1, caractérisé par le fait que l'émulsionnant E/H de ladite phase $H_2$ est choisi parmi les diméthicone copolyols et leurs esters.

3. Procédé selon la revendication 1 ou 2, caractérisé par le fait que ledit filtre hydrophile est choisi parmi les dérivés de la benzophénone, les dérivés de l'acide p-aminobenzoïque, les dérivés du camphre ou encore parmi les dérivés de benzimidazole.

4. Procédé selon la revendication 3, caractérisé par le fait que ledit filtre hydrophile est choisi parmi les dérivés du camphre.

5. Procédé selon la revendication 4, caractérisé par le fait que ledit filtre hydrophile est l'acide benzène 1,4-di(3-méthylidène-10-camphosulfonique).

6. Procédé selon la revendication 3, caractérisé par le fait que ledit filtre hydrophile est choisi parmi les dérivés de benzimidazole.

7. Procédé selon la revendication 6, caractérisé par le fait que ledit filtre hydrophile est l'acide 2-phénylbenzimidazole-5-sulfonique.

8. Procédé selon l'une quelconque des revendications 1 à 7, caractérisé par le fait que le filtre hydrophile est présent dans la composition finale à une teneur pouvant aller de 0,1 à 20 %, en poids, par rapport au poids total de la composition.

9. Procédé selon la revendication 8, caractérisé par le fait que ladite teneur va de 0,2 à 10 %, en poids, par rapport au poids total de la composition.

10. Procédé selon l'une quelconque des revendications 1 à 9, caractérisé par le fait que ledit filtre lipophile est choisi parmi les dérivés du dibenzoyméthane, les dérivés de benzimidazole, les dérivés cinnamiques, les dérivés salicyliques, les dérivés du camphre, les dérivés de triazine, les dérivés de la benzophénone, les dérivés de $\beta,\beta$-diphénylacrylate, les dérivés de l'acide p-aminobenzoïque, les polymères filtres et les silicones filtres.

11. Procédé selon la revendication 10, caractérisé par le fait que ledit filtre lipophile est choisi parmi les dérivés du dibenzoylméthane.

12. Procédé selon la revendication 11, caractérisé par le fait que ledit filtre lipophile est le 4-tert-butyl-4'-méthoxydibenzoylméthane.

13. Procédé selon la revendication 10, caractérisé par le fait que ledit filtre lipophile est choisi parmi les dérivés de $\beta,\beta$-diphénylacrylate.

14. Procédé selon la revendication 13, caractérisé par le fait que ledit filtre lipophile est l'$\alpha$-cyano-$\beta,\beta$-diphénylacrylate de 2-éthylhexyle.

15. Procédé selon l'une quelconque des revendications 1 à 14, caractérisé par le fait que ledit filtre lipophile est présent dans la composition finale à une teneur pouvant aller de 0,5 à 30 %, en poids, par rapport au poids total de la composition.

16. Procédé selon la revendication 15, caractérisé par le fait que ladite teneur va de 0,2 à 20 %, en poids, par rapport au poids total de la composition.

17. Procédé selon l'une quelconque des revendications 1 à 16, caractérisé par le fait que la phase $H_2$ comprend une silicone volatile.

18. Procédé selon la revendication 17, caractérisé par le fait que ladite silicone est une silicone volatile.

**19.** Procédé selon la revendication 18, caractérisé par le fait que ladite silicone volatile est présente dans la composition finale à une teneur allant de 1 à 20 %, en poids, par rapport au poids total de la composition.

**20.** Procédé selon la revendication 19, caractérisé par le fait que ladite teneur va de 2 à 10 %, en poids, par rapport au poids total de la composition.

**21.** Procédé selon l'une quelconque des revendications 1 à 20, caractérisé par le fait que la phase $H_1$ est exempte de filtre.

**22.** Procédé selon l'une quelconque des revendications 1 à 21, caractérisé par le fait que la phase aqueuse est exempte de filtre.

**23.** Procédé selon l'une quelconque des revendications 1 à 22, caractérisé par le fait que la phase $H_2$ comprend tous les filtres contenus dans la composition finale.

**24.** Composition cosmétique et/ou dermatologique susceptible d'être obtenue par le procédé défini à l'une quelconque des revendications 1 à 23, caractérisée par le fait qu'elle comprend :

i) une phase aqueuse continue dispersante,

ii) une première phase huileuse $H_1$ dispersée discontinue,

iii) une deuxième phase huileuse $H_2$ dispersée discontinue,

iv) au moins un émulsionnant H/E,

v) au moins un émulsionnant E/H,

vi) au moins un filtre lipophile,

vii) au moins un filtre hydrophile,

lesdites première et deuxième phases huileuses $H_1$ et $H_2$ étant incompatibles entre elles, les globules constituant ladite phase huileuse $H_1$ ayant une taille moyenne différente des globules constituant ladite phase huileuse H2.

**25.** Composition selon la revendication 24, caractérisée par le fait que l'émulsionnant E/H de ladite phase $H_2$ est choisi parmi les diméthicone copolyols et leurs esters.

**26.** Composition selon la revendication 24 ou 25, caractérisée par le fait que ledit filtre hydrophile est choisi parmi les dérivés de la benzophénone, les dérivés de l'acide p-aminobenzoïque, les dérivés du camphre ou encore parmi les dérivés de benzimidazole.

**27.** Composition selon la revendication 26, caractérisée par le fait que ledit filtre hydrophile est choisi parmi les dérivés du camphre.

**28.** Composition selon la revendication 27, caractérisée par le fait que ledit filtre hydrophile est l'acide benzène 1,4-di(3-méthylidène-10-camphosulfonique).

**29.** Composition selon la revendication 26, caractérisée par le fait que ledit filtre hydrophile est choisi parmi les dérivés de benzimidazole.

**30.** Composition selon la revendication 29, caractérisée par le fait que ledit filtre hydrophile est l'acide 2-phénylbenzimidazole-5-sulfonique.

**31.** Composition selon l'une quelconque des revendications 24 à 30, caractérisée par le fait que le filtre hydrophile est présent dans la composition à une teneur pouvant aller de 0,1 à 20 %, en poids, par rapport au poids total de la composition.

**32.** Composition selon la revendication 31, caractérisée par le fait que ladite teneur va de 0,2 à 10 %, en poids, par rapport au poids total de la composition.

**33.** Composition selon l'une quelconque des revendications 24 à 32, caractérisée par le fait que ledit filtre lipophile est choisi parmi les dérivés du dibenzoyméthane, les dérivés de benzimidazole, les dérivés cinnamiques, les dérivés salicyliques, les dérivés du camphre, les dérivés de triazine, les dérivés de la benzophénone, les dérivés de $\beta,\beta$-diphénylacrylate, les dérivés de l'acide p-aminobenzoïque, les polymères filtres et silicones filtres.

**34.** Composition selon la revendication 33, caractérisée par le fait que ledit filtre lipophile est choisi parmi les dérivés du dibenzoylméthane.

**35.** Composition selon la revendication 34, caractérisée par le fait que ledit filtre lipophile est le 4-tert-butyl-4'-méthoxydibenzoylméthane.

**36.** Composition selon la revendication 33, caractérisée par le fait que ledit filtre lipophile est choisi parmi les dérivés de $\beta,\beta$-diphénylacrylate.

**37.** Composition selon la revendication 36, caractérisée par le fait que ledit filtre lipophile est l'$\alpha$-cyano-$\beta,\beta$-diphényla-crylate de 2-éthylhexyle.

**38.** Composition selon l'une quelconque des revendications 24 à 37, caractérisée par le fait que ledit filtre lipophile est présent dans la composition à une teneur pouvant aller de 0,5 à 30 %, en poids, par rapport au poids total de la composition.

**39.** Composition selon la revendication 38, caractérisée par le fait que ladite teneur va de 0,2 à 20 %, en poids, par rapport au poids total de la composition.

**40.** Composition selon l'une quelconque des revendications 24 à 39, caractérisée par le fait que la phase $H_2$ comprend une silicone.

**41.** Composition selon la revendication 40, caractérisée par le fait que ladite silicone est une silicone volatile.

**42.** Composition selon la revendication 40 ou 41, caractérisée par le fait que ladite silicone est présente dans la composition à une teneur allant de 1 à 20 %, en poids, par rapport au poids total de la composition.

**43.** Composition selon la revendication 42, caractérisée par le fait que ladite teneur va de 2 à 10 %, en poids, par rapport au poids total de la composition.

**44.** Composition selon l'une quelconque des revendications 24 à 43, caractérisée par le fait que ladite composition comprend en outre des nanopigments d'oxyde métallique.

**45.** Procédé de traitement cosmétique pour la protection de la peau et/ou des cheveux contre le rayonnement ultraviolet, en particulier le rayonnement solaire, caractérisé par le fait qu'il consiste à appliquer sur ces derniers une quantité efficace d'une composition telle que définie à l'une quelconque des revendications 24 à 44.

**46.** Utilisation de la composition définie à l'une quelconque des revendications 24 à 44 comme, ou pour la fabrication de, compositions cosmétiques pour la protection de la peau et/ou des cheveux contre le rayonnement ultraviolet, en particulier le rayonnement solaire.

**Claims**

**1.** Process for the preparation of a composition comprising, in a cosmetically and/or dermatologically acceptable vehicle of mixed oil-water type, at least one lipophilic screening agent and at least one hydrophilic screening agent, characterized in that it consists essentially in introducing, in an oil-in-water emulsion obtained by mixing i) an aqueous phase A and ii) an oily phase $O_1$ comprising an O/W emulsifying agent, an oily phase $O_2$ comprising a W/O emulsifying agent, the said lipophilic screening agent and the said hydrophilic screening agent, the said oily phases $O_1$ and $O_2$ being mutually incompatible.

2. Process according to Claim 1, characterized in that the W/O emulsifying agent of the said phase $O_2$ is chosen from dimethicone copolyols and their esters.

3. Process according to Claim 1 or 2, characterized in that the said hydrophilic screening agent is chosen from benzophenone derivatives, p-aminobenzoic acid derivatives, camphor derivatives and benzimidazole derivatives.

4. Process according to Claim 3, characterized in that the said hydrophilic screening agent is chosen from camphor derivatives.

5. Process according to Claim 4, characterized in that the said hydrophilic screening agent is benzene-1,4-di(3-methylidene-10-camphorsulphonic) acid.

6. Process according to Claim 3, characterized in that the said hydrophilic screening agent is chosen from benzimidazole derivatives.

7. Process according to Claim 6, characterized in that the said hydrophilic screening agent is 2-phenylbenzimidazole-5-sulphonic acid.

8. Process according to any one of Claims 1 to 7, characterized in that the hydrophilic screening agent is present in the final composition in a content which may range from 0.1 to 20% by weight relative to the total weight of the composition.

9. Process according to Claim 8, characterized in that the said content ranges from 0.2 to 10% by weight relative to the total weight of the composition.

10. Process according to any one of Claims 1 to 9, characterized in that the said lipophilic screening agent is chosen from dibenzoylmethane derivatives, benzimidazole derivatives; cinnamic derivatives, salicylic derivatives, camphor derivatives, triazine derivatives, benzophenone derivatives, $\beta,\beta$-diphenylacrylate derivatives, p-aminobenzoic acid derivatives, screening polymers and screening silicones.

11. Process according to Claim 10, characterized in that the said lipophilic screening agent is chosen from dibenzoylmethane derivatives.

12. Process according to Claim 11, characterized in that the said lipophilic screening agent is 4-tert-butyl-4'-methoxydibenzoylmethane.

13. Process according to Claim 10, characterized in that the said lipophilic screening agent is chosen from $\beta,\beta$-diphenylacrylate derivatives.

14. Process according to Claim 13, characterized in that the said lipophilic screening agent is 2-ethylhexyl $\alpha$-cyano-$\beta,\beta$-diphenylacrylate.

15. Process according to any one of Claims 1 to 14, characterized in that the said lipophilic screening agent is present in the final composition in a content which may range from 0.5 to 30% by weight relative to the total weight of the composition.

16. Process according to Claim 15, characterized in that the said content ranges from 0.2 to 20% by weight relative to the total weight of the composition.

17. Process according to any one of Claims 1 to 16, characterized in that the phase $O_2$ comprises a silicone.

18. Process according to Claim 17, characterized in that the said silicone is a volatile silicone.

19. Process according to Claim 18, characterized in that the said volatile silicone is present in the final composition in a content ranging from 1 to 20% by weight relative to the total weight of the composition.

20. Process according to Claim 19, characterized in that the said content ranges from 2 to 10% by weight relative to the total weight of the composition.

**21.** Process according to any one of Claims 1 to 20, characterized in that the phase $O_1$ is free of screening agent.

**22.** Process according to any one of Claims 1 to 21, characterized in that the aqueous phase is free of screening agent.

**23.** Process according to any one of Claims 1 to 22, characterized in that the phase $O_2$ comprises all the screening agents contained in the final composition.

**24.** Cosmetic and/or dermatological composition which may be obtained by the process defined in any one of Claims 1 to 23, characterized in that it comprises:

    i) a continuous aqueous dispersing phase,
    ii) a first discontinuous oily dispersed phase $O_1$,
    iii) a second discontinuous oily dispersed phase $O_2$,
    iv) at least one O/W emulsifying agent,
    v) at least one W/O emulsifying agent,
    vi) at least one lipophilic screening agent,
    vii) at least one hydrophilic screening agent;

the said first and second oily phases $O_1$ and $O_2$ being mutually incompatible, the globules constituting the said oily phase $O_1$ having an average size which is different from the globules constituting the said oily phase $O_2$.

**25.** Composition according to Claim 24, characterized in that the W/O emulsifying agent of the said phase $O_2$ is chosen from dimethicone copolyols and their esters.

**26.** Composition according to Claim 24 or 25, characterized in that the said hydrophilic screening agent is chosen from benzophenone derivatives, p-aminobenzoic acid derivatives, camphor derivatives and benzimidazole derivatives.

**27.** Composition according to Claim 26, characterized in that the said hydrophilic screening agent is chosen from camphor derivatives.

**28.** Composition according to Claim 27, characterized in that the said hydrophilic screening agent is benzene-1,4-di(3-methylidene-10-camphorsulphonic) acid.

**29.** Composition according to Claim 26, characterized in that the said hydrophilic screening agent is chosen from benzimidazole derivatives.

**30.** Composition according to Claim 29, characterized in that the said hydrophilic screening agent is 2-phenylbenzimidazole-5-sulphonic acid.

**31.** Composition according to any one of Claims 24 to 30, characterized in that the hydrophilic screening agent is present in the composition in a content which may range from 0.1 to 20% by weight relative to the total weight of the composition.

**32.** Composition according to Claim 31, characterized in that the said content ranges from 0.2 to 10% by weight relative to the total weight of the composition.

**33.** Composition according to any one of Claims 24 to 32, characterized in that the said lipophilic screening agent is chosen from dibenzoylmethane derivatives, benzimidazole derivatives, cinnamic derivatives, salicylic derivatives, camphor derivatives, triazine derivatives, benzophenone derivatives, $\beta,\beta$-diphenylacrylate derivatives, p-aminobenzoic acid derivatives, screening polymers and screening silicones.

**34.** Composition according to Claim 33, characterized in that the said lipophilic screening agent is chosen from dibenzoylmethane derivatives.

**35.** Composition according to Claim 34, characterized in that the said lipophilic screening agent is 4-tert-butyl-4'-methoxydibenzoylmethane.

**36.** Composition according to Claim 33, characterized in that the said lipophilic screening agent is chosen from $\beta,\beta$-

diphenylacrylate derivatives.

37. Composition according to Claim 36, characterized in that the said lipophilic screening agent is 2-ethylhexyl $\alpha$-cyano-$\beta$,$\beta$-diphenylacrylate.

38. Composition according to any one of Claims 24 to 37, characterized in that the said lipophilic screening agent is present in the composition in a content which may range from 0.5 to 30% by weight relative to the total weight of the composition.

39. Composition according to Claim 38, characterized in that the said content ranges from 0.2 to 20% by weight relative to the total weight of the composition.

40. Composition according to any one of Claims 24 to 39, characterized in that the phase $O_2$ comprises a silicone.

41. Composition according to Claim 40, characterized in that the said silicone is a volatile silicone.

42. Composition according to Claim 40 or 41, characterized in that the said silicone is present in the composition in a content ranging from 1 to 20% by weight relative to the total weight of the composition.

43. Composition according to Claim 42, characterized in that the said content ranges from 2 to 10% by weight relative to the tocal weight of the composition.

44. Composition according to any one of Claims 24 to 43, characterized in that the said composition also comprises metal oxide nanopigments.

45. Cosmetic treatment process for protecting the skin and/or the hair against ultraviolet radiation, in particular solar radiation, characterized in that it consists in applying an effective amount of a composition as defined in any one of Claims 24 to 44 to the skin and/or the hair.

46. Use of the composition defined in any one of Claims 24 to 44 as, or for the manufacture of, cosmetic compositions for protecting the skin and/or the hair against ultraviolet radiation, in particular solar radiation.

**Patentansprüche**

1. Verfahren zur Herstellung einer Zusammensetzung, die in einem kosmetisch und/oder dermatologisch akzeptablen Träger vom Öl-Wasser-Mischtyp mindestens ein lipophiles Filter und mindestens ein hydrophiles Filter enthält, dadurch gekennzeichnet, daß es im wesentlichen darin besteht, zu einer Öl-in-Wasser-Emulsion, die durch Vermischen i) einer wäßrigen Phase A und ii) einer Ölphase $H_1$, die einen O/W-Emulgator enthält, hergestellt wird, eine Ölphase $H_2$ zu geben, die einen W/O-Emulgator, das obengenannte lipophile Filter und das obengenannte hydrophile Filter enthält, wobei die Ölphase $H_1$ und und die Ölphase $H_2$ untereinander inkompatibel sind.

2. Verfahren nach Anspruch 1, dadurch gekennzeichnet, daß der W/O-Emulgator der Phase $H_2$ unter Dimethicon-Copolyolen und deren Estern ausgewählt wird.

3. Verfahren nach Anspruch 1 oder 2, dadurch gekennzeichnet, daß das hydrophile Filter unter Benzophenon-Derivaten, p-Aminobenzoesäure-Derivaten, Campher-Derivaten und Benzimidazol-Derivaten ausgewählt wird.

4. Verfahren nach Anspruch 3, dadurch gekennzeichnet, daß das hydrophile Filter unter Campher-Derivaten ausgewählt wird.

5. Verfahren nach Anspruch 4, dadurch gekennzeichnet, daß es sich bei dem hydrophilen Filter um 1,4-Benzoldi-(3-methyliden-10-camphersulfonsäure) handelt.

6. Verfahren nach Anspruch 3, dadurch gekennzeichnet, daß das hydrophile Filter unter Benzimidazol-Derivaten ausgewählt wird.

7. Verfahren nach Anspruch 6, dadurch gekennzeichnet, daß es sich bei dem hydrophilen Filter um 2-Phenylbenzimidazol-5-sulfonsäure handelt.

8. Verfahren nach einem der Ansprüche 1 bis 7, dadurch gekennzeichnet, daß das hydrophile Filter in der fertigen Zusammensetzung in einem Anteil enthalten ist, der 0,1 bis 20 Gew.-%, bezogen auf das Gesamtgewicht der Zusammensetzung, betragen kann.

9. Verfahren nach Anspruch 8, dadurch gekennzeichnet, daß der Anteil im Bereich von 0,2 bis 10 Gew.-%, bezogen auf das Gesamtgewicht der Zusammensetzung, liegt.

10. Verfahren nach einem der Ansprüche 1 bis 9, dadurch gekennzeichnet, daß das lipophile Filter unter Dibenzoylmethan-Derivaten, Benzimidazol-Derivaten, Zimtsäure-Derivaten, Salicylsäure-Derivaten, Campher-Derivaten, Triazin-Derivaten, Benzophenon-Derivaten, $\beta,\beta$-Diphenylacrylat-Derivaten, p-Aminobenzoesäure-Derivaten, Polymerfiltern und Siliconfiltern ausgewählt wird.

11. Verfahren nach Anspruch 10, dadurch gekennzeichnet, daß das lipophile Filter unter Dibenzoylmethan-Derivaten ausgewählt wird.

12. Verfahren nach Anspruch 11, dadurch gekennzeichnet, daß es sich bei dem lipophilen Filter um 4-*tert*.-Buty-4'-methoxydibenzoylmethan handelt.

13. Verfahren nach Anspruch 10, dadurch gekennzeichnet, daß das lipophile Filter unter $\beta,\beta$-Diphenylacrylat-Derivaten ausgewählt wird.

14. Verfahren nach Anspruch 13, dadurch gekennzeichnet, daß es sich bei dem lipophilen Filter um den $\alpha$-Cyano-$\beta,\beta$-diphenylacrylsäure-2-ethylhexylester handelt.

15. Verfahren nach einem der Ansprüche 1 bis 14, dadurch gekennzeichnet, daß das lipophile Filter in der fertigen Zusammensetzung in einem Anteil enthalten ist, der 0,5 bis 30 Gew.-%, bezogen auf das Gesamtgewicht der Zusammensetzung, betragen kann.

16. Verfahren nach Anspruch 15, dadurch gekennzeichnet, daß der Anteil im Bereich von 0,2 bis 20 Gew.-%, bezogen auf das Gesamtgewicht der Zusammensetzung, liegt.

17. Verfahren nach einem der Ansprüche 1 bis 16, dadurch gekennzeichnet, daß die Phase $H_2$ ein flüchtiges Silicon enthält.

18. Verfahren nach Anspruch 17, dadurch gekennzeichnet, daß das Silicon ein flüchtiges Silicon ist.

19. Verfahren nach Anspruch 18, dadurch gekennzeichnet, daß das flüchtige Silicon in der fertigen Zusammensetzung in einem Anteil von 1 bis 20 Gew.-%, bezogen auf das Gesamtgewicht der Zusammensetzung, enthalten ist.

20. Verfahren nach Anspruch 19, dadurch gekennzeichnet, daß der Anteil 2 bis 10 Gew.-%, bezogen auf das Gesamtgewicht der Zusammensetzung, beträgt.

21. Verfahren nach einem der Ansprüche 1 bis 20, dadurch gekennzeichnet, daß die Phase $H_1$ kein Filter enthält.

22. Verfahren nach einem der Ansprüche 1 bis 21, dadurch gekennzeichnet, daß die wäßrige Phase kein Filter enthält.

23. Verfahren nach einem der Ansprüche 1 bis 22, dadurch gekennzeichnet, daß die Phase $H_2$ alle in der fertigen Zusammensetzung enthaltenen Filter enthält.

24. Kosmetische und/oder dermatologische Zusammensetzung, die nach dem in einem der Ansprüche 1 bis 23 definierten Verfahren hergestellt werden kann, dadurch gekennzeichnet, daß sie enthält:

> i) eine kontinuierliche dispergierende wäßrige Phase,
> ii) eine erste diskontinuierliche dispergierte Ölphase $H_1$,
> iii) eine zweite diskontinuierliche dispergierte Ölphase $H_2$,
> iv) mindestens einen O/W-Emulgator,
> v) mindestens einen W/O-Emulgator,
> vi) mindestens ein lipophiles Filter,

vii) mindestens ein hydrophiles Filter,

wobei die erste Ölphase $H_1$ und die zweite Ölphase $H_2$ untereinander inkompatibel sind, wobei die Kügelchen, die die Ölphase $H_1$ bilden, eine mittlere Größe aufweisen, die von der mittleren Größe der Kügelchen, die die Ölphase $H_2$ bilden, verschieden ist.

25. Zusammensetzung nach Anspruch 24, dadurch gekennzeichnet, daß der W/O-Emulgator der Phase $H_2$ unter Dimethicon-Copolyolen und deren Estern ausgewählt ist.

26. Zusammensetzung nach Anspruch 24 oder 25, dadurch gekennzeichnet, daß das hydrophile Filter unter Benzophenon-Derivaten, p-Aminobenzoesäure-Derivaten, Campher-Derivaten und Benzimidazol-Derivaten ausgewählt ist.

27. Zusammensetzung nach Anspruch 26, dadurch gekennzeichnet, daß das hydrophile Filter unter Campher-Derivaten ausgewählt ist.

28. Zusammensetzung nach Anspruch 27, dadurch gekennzeichnet, daß es sich bei dem hydrophilen Filter um 1,4-Benzoldi-(3-methyliden-10-camphersulfonsäure) handelt.

29. Zusammensetzung nach Anspruch 26, dadurch gekennzeichnet, daß das hydrophile Filter unter Benzimidazol-Derivaten ausgewählt ist.

30. Zusammensetzung nach Anspruch 29, dadurch gekennzeichnet, daß es sich bei dem hydrophilen Filter um 2-Phenylbenzimidazol-5-sulfonsäure handelt.

31. Zusammensetzung nach einem der Ansprüche 24 bis 30, dadurch gekennzeichnet, daß das hydrophile Filter in der Zusammensetzung in einem Anteil enthalten ist, der 0,1 bis 20 Gew.-%, bezogen auf das Gesamtgewicht der Zusammensetzung, betragen kann.

32. Zusammensetzung nach Anspruch 31, dadurch gekennzeichnet, daß der Anteil im Bereich von 0,2 bis 10 Gew.-%, bezogen auf das Gesamtgewicht der Zusammensetzung, liegt.

33. Zusammensetzung nach einem der Ansprüche 24 bis 32, dadurch gekennzeichnet, daß das lipophile Filter unter Dibenzoylmethan-Derivaten, Benzimidazol-Derivaten, Zimtsäure-Derivaten, Salicylsäure-Derivaten, Campher-Derivaten, Triazin-Derivaten, Benzophenon-Derivaten, $\beta,\beta$-Diphenylacrylat-Derivaten, p-Aminobenzoesäure-Derivaten, Polymerfiltern und Siliconfiltern ausgewählt ist.

34. Zusammensetzung nach Anspruch 33, dadurch gekennzeichnet, daß das lipophile Filter unter Dibenzoylmethan-Derivaten ausgewählt ist.

35. Zusammensetzung nach Anspruch 34, dadurch gekennzeichnet, daß es sich bei dem lipophilen Filter um 4-*tert*.-Butyl-4'-methoxydibenzoylmethan handelt.

36. Zusammensetzung nach Anspruch 33, dadurch gekennzeichnet, daß das lipophile Filter unter den $\beta,\beta$-Diphenylacrylat-Derivaten ausgewählt ist.

37. Zusammensetzung nach Anspruch 36, dadurch gekennzeichnet, daß es sich bei dem lipophilen Filter um den $\alpha$-Cyano-$\beta,\beta$-diphenylacrylsäure-2-ethylhexylester handelt.

38. Zusammensetzung nach einem der Ansprüche 24 bis 37, dadurch gekennzeichnet, daß das lipophile Filter in der Zusammensetzung in einem Anteil enthalten ist, der 0,5 bis 30 Gew.-%, bezogen auf das Gesamtgewicht der Zusammensetzung, betragen kann.

39. Zusammensetzung nach Anspruch 38, dadurch gekennzeichnet, daß der Anteil im Bereich von 0,2 bis 20 Gew.-%, bezogen auf das Gesamtgewicht der Zusammensetzung, liegt.

40. Zusammensetzung nach einem der Ansprüche 24 bis 39, dadurch gekennzeichnet, daß die Phase $H_2$ ein Silicon enthält.

**41.** Zusammensetzung nach Anspruch 40, dadurch gekennzeichnet, daß das Silicon ein flüchtiges Silicon ist.

**42.** Zusammensetzung nach Anspruch 40 oder 41, dadurch gekennzeichnet, daß das Silicon in der Zusammensetzung in einem Anteil von 1 bis 20 Gew.-%, bezogen auf das Gesamtgewicht der Zusammensetzung, enthalten ist.

**43.** Zusammensetzung nach Anspruch 42, dadurch gekennzeichnet, daß der Anteil im Bereich von 2 bis 10 Gew.-%, bezogen auf das Gesamtgewicht der Zusammensetzung, liegt.

**44.** Zusammensetzung nach einem der Ansprüche 24 bis 43, dadurch gekennzeichnet, daß die Zusammensetzung außerdem Metalloxid-Nanopigmente enthält.

**45.** Verfahren zur kosmetischen Behandlung zum Schutz der Haut und/oder der Haare vor UV-Strahlung, insbesondere Sonnenlicht, dadurch gekennzeichnet, daß es darin besteht, auf die Haut und/oder die Haare eine wirksame Menge einer wie in einem der Ansprüche 24 bis 44 definierten Zusammensetzung aufgetragen wird.

**46.** Verwendung der in einem der Ansprüche 24 bis 44 definierten Zusammensetzung als kosmetische Zusammensetzungen oder für die Herstellung von kosmetischen Zusammensetzungen für den Schutz der Haut und/oder der Haare vor UV-Strahlung, insbesondere Sonnenlicht.